# EUROPEAN PATENT APPLICATION

(11) **EP 2 468 233 A1**
(43) Date of publication of application: **27.06.2012**
(21) Application number: 11195270.1
(22) Date of filing: 22.12.2011
(51) Int. Cl.: A61J 1/03, A61J 7/04

(54) **Color change time indicator for packaging system**

(30) Priority: 22.12.2010 US 201013426204
(71) Applicant: Purdue Pharma LP, Stamford, CT 06901 (US)
(72) Inventor: Steiner, LaDonna, Fairfield, CT Connecticut 06430 (US)
(74) Representative: Maiwald Patentanwalts GmbH

(57) **Abstract**

Disclosed in certain embodiments is a pharmaceutical packaging system comprising: a receptacle (2) comprising an openable seal (4) for accessing the interior of the receptacle (2); a pharmaceutical dosage form (3) contained in the receptacle (2); and an activatable color changing material (5), wherein upon activation, the material (5) changes color after a predetermined time.

## Description

### FIELD OF THE INVENTION

The present invention is directed to color-change time indicators for pharmaceutical packaging systems. The indicators can be utilized for a variety of uses, e.g., to indicate the end of a dosing interval or when it is safe to resume an activity such as the operation of machinery.

### BACKGROUND OF THE INVENTION

Pharmaceuticals have greatly benefited society in many ways such as eradicating or reducing the incidence of disease states and managing the symptoms of debilitating conditions.

In many instances, patients do not receive the full benefit of their therapy due to non-compliance with a prescribed dosing regimen. A common non-compliance issue with patients is administering the drug at incorrect dosing intervals. For example, if a drug is intended to be prescribed every eight hours, a patient may not achieve the proper plasma drug level if the drug is administered three times daily but at random intervals. Such incorrect dosing can result in toxic or sub-therapeutic plasma drug levels throughout the day.

Also, even with modem advancements in medicine, drug therapy usually cannot specifically target the intended site of action, but instead also results in unintended side effects. A common side effect of many drugs is sedation and decreased central nervous system function for a period of time after administration. Patients are typically warned not to participate in certain activities until the sedation subsides. Unfortunately, many patients initiate these activities prior to the end of this time period. This can result in serious problems, such as patients driving or operating machinery while under the influence of prescription medicine.

In instances where sedation is the intended pharmacologic effect (e.g., in the treatment of insomnia), a potential side effect is a "hangover" effect, where the patient has residual sedation upon awakening in the morning. This problem can be compounded when a patient takes the sedative at a time later than prescribed such that the intended dosing interval overlaps with the initiation of activities upon awakening of the patient.

There exists a need in the art for an apparatus and method for indicating to a patient or caregiver that a specific time period has elapsed in order to indicate that a subsequent dose is due and/or to indicate that it is safe to resume certain activities (e.g., driving).

All references described herein are hereby incorporated by reference in their entireties for all purposes.

### OBJECTS AND SUMMARY OF THE INVENTION

It is an object of certain embodiments of the invention to provide a system for indicating the passage of a specified time after opening a pharmaceutical packaging unit.

It is an object of certain embodiments of the invention to provide a system for indicating the passage of a specified time after unpacking of a pharmaceutical dosage form.

It is an object of certain embodiments of the invention to provide a system for indicating the passage of a specified time after administration of a pharmaceutical dosage form.

It is an object of certain embodiments of the invention to provide a system for indicating the passage of a specified time after administration of a pharmaceutical dosage form, which can be clearly and unambiguously recognized by the patient.

It is an object of certain embodiments of the invention to provide a system to promote patient compliance to a pharmaceutical dosage regimen.

It is an object of certain embodiments of the invention to provide a system to indicate that a patient can resume certain activities (e.g., driving) after the passage of a specified time after administration of a pharmaceutical dosage form.

It is an object of certain embodiments of the invention to increase public safety by decreasing the incidence of certain activities being performed by patients under the influence of pharmacological treatment.

It is an object of certain embodiments of the invention to increase patient compliance to pharmacological treatments and dosing regimens (e.g., for treating insomnia).

It is an object of certain embodiments of the invention to indicate any damage to a pharmaceutical packaging unit which may occur during transport or storage of said unit.

The above objects and others, are met by one or more aspects of the present invention, which in certain embodiments is directed to a pharmaceutical packaging system comprising: a receptacle comprising an openable seal for accessing the interior of the receptacle; a pharmaceutical dosage form contained in the receptacle; and an activatable color-changing material, wherein upon activation, the material changes color upon the lapsing of a predetermined time. Advantageously, in certain embodiments the color-changing material is activated upon opening of the seal.

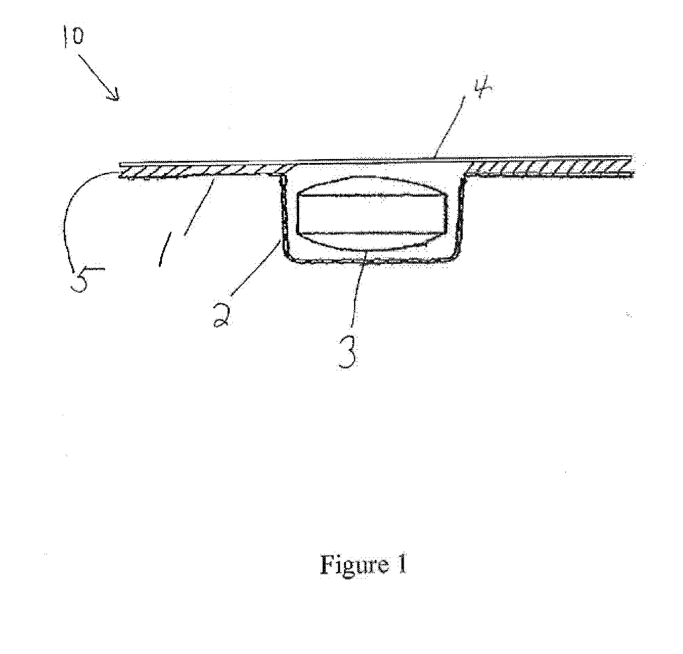

In certain embodiments exemplified by Figure 1, the present invention is directed to a pharmaceutical packaging system comprising: a substrate comprising a base surface; a blister concave to the base surface, wherein the blister is adapted to contain a pharmaceutical dosage form; a removable surface layered over the base surface; and an activatable color-changing material between the base surface and the removable surface; wherein upon separation of the removable surface from the base surface, the material is activated, and such activation results in a visually discernible change in color of the material upon the lapsing of a predetermined time period.

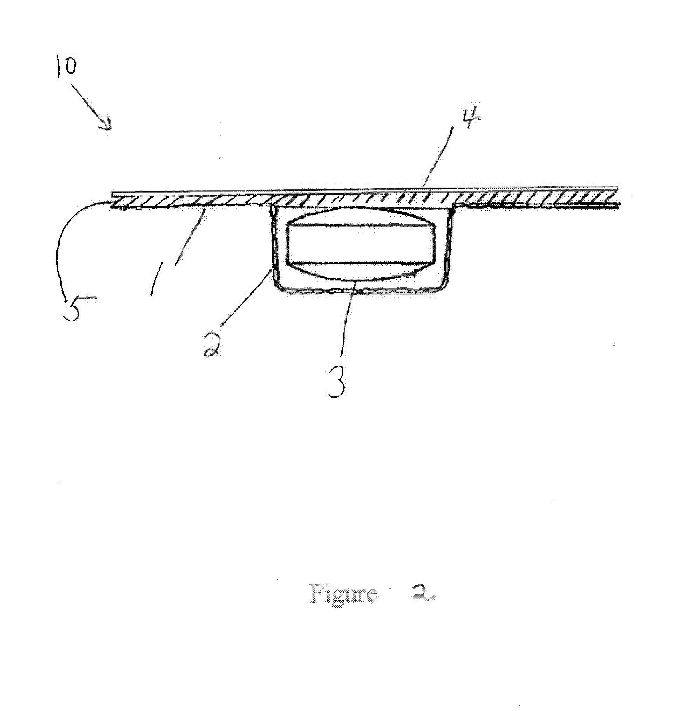

In certain embodiments exemplified by Figure 2, the present invention is directed to a pharmaceutical packaging system comprising an activatable color-changing material between the base surface and the removable surface, wherein the material is also layered beneath the removable surface and above the blister concave.

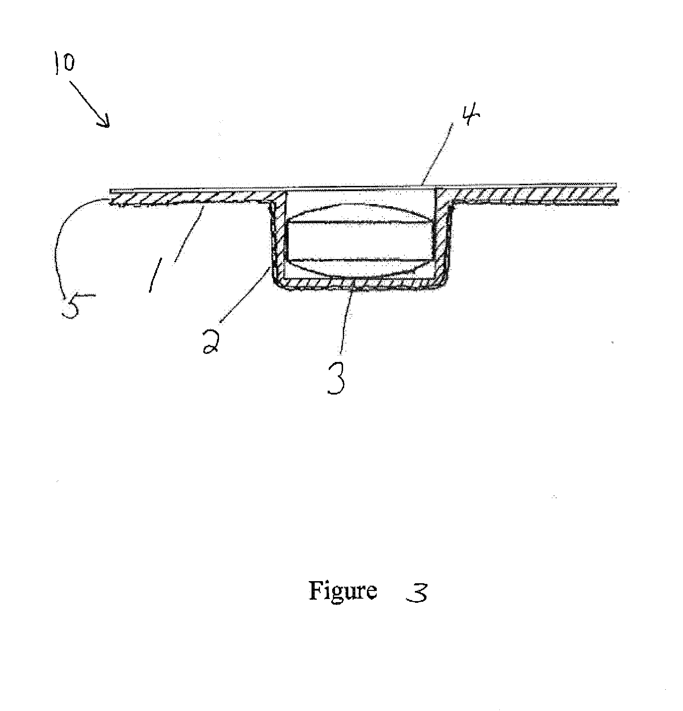

In certain embodiments exemplified by Figure 3, the present invention is directed to a pharmaceutical packaging system comprising an activatable color-changing material between the base surface and the removable surface, wherein the material is also layered over the base surface in the blister concave.

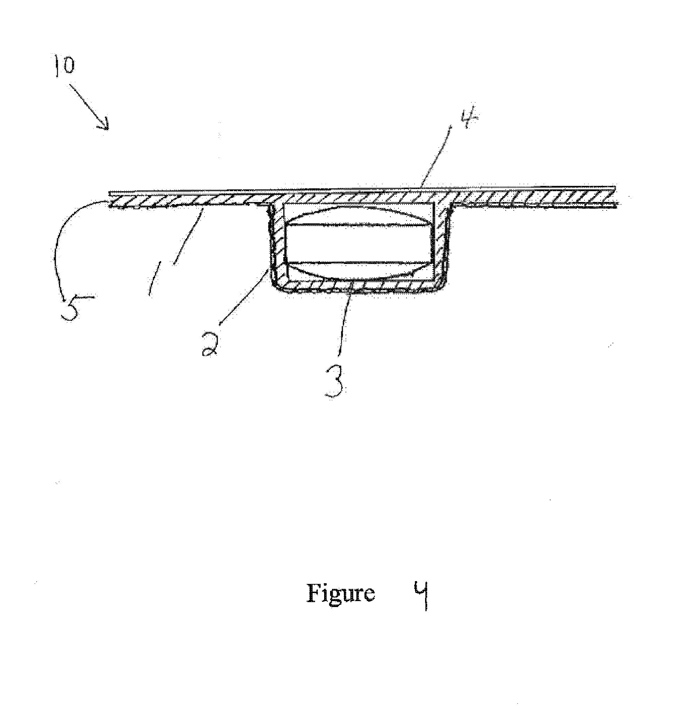

In certain embodiments exemplified by Figure 4, the present invention is directed to a pharmaceutical packaging system comprising an activatable color-changing material between the base surface and the removable surface, wherein the material is also layered beneath the removable surface above the blister concave and wherein the material is layered over the base surface in the blister concave, thereby enclosing the pharmaceutical dosage form.

In certain embodiments, the present invention is directed to a pharmaceutical packaging system for use in the treatment of a patient suffering from a condition, wherein the packaging system comprises a substrate comprising a base surface; a blister concave to the base surface, wherein the blister contains a pharmaceutical dosage form comprising a drug effective to treat the condition; a removable surface layered over the base surface; and an activatable color-changing material. In certain embodiments, the color-changing material is layered according to any one of the above recited embodiments exemplified by Figures 1 to 4; wherein upon separation of the removable surface from the base surface, the material is activated, and such activation results in a visually discernible change in color of the material upon the lapsing of a predetermined time period.

The visually discernible color change upon the lapsing of the predetermined time period indicates the end of said predetermined period. Said predetermined period can be, for example, the dosing interval, the interval of pharmaceutical activity of the active agent, and/or the interval after which the patient is allowed to resume certain activities like driving or alcohol uptake.

In one aspect, the color change indicates that the patient is substantially no longer under the influence of the active agent.

In another aspect the color change indicates that the patient is allowed to administer an additional or subsequent dosage form.

In another aspect the color change indicates that the patient is allowed to initiate driving acivities.

In another aspect the color change indicates that the patient is instructed to check his or her condition to maintain or adapt the dosage.

In even another aspect, the color-change can be an indicator for a damage to the packaging system, e.g. an unintentional damage during transport.

The patient treated with the dosage form contained in the pharmaceutical packaging system may suffer from any condition requiring administration of a drug, in particular of a drug which exerts its function over a predetermined peroid of time or which has to be taken in certain time intervals (e.g., in a dosing regimen or during long-term treatment). In certain embodiments, the condition is pain. In other embodiments, the condition is insomnia.

In certain embodiments, the present invention is directed to a method of packaging a pharmaceutical dosage form (e.g., a dosage form comprising an opioid or a sedative/hypnotic as active agent) comprising packaging the dosage form with a packaging system comprising the activatable color-changing material, wherein removal of the dosage form from the packaging system serves to activate the activatable color-changing material, and wherein the activatable color-changing material exhibits a visually discernible color change upon the lapsing of a predetermined time period after said activation.

In certain embodiments, the present invention is directed to a kit comprising: (i) a pharmaceutical dosage form (e.g., a dosage form comprising an opioid or sedative/hypnotic as active agent); and (ii) an activatable color-changing material, wherein upon activation, the activatable color-changing material exhibits a visually discernible color change after a predetermined time period has elapsed after said activation. In certain embodiments, activation occurs when the kit is compromised or when the a pharmaceutical dosage form is removed from the kit. The kit may optionally further comprise indicia instructing the patient that the color change occurring upon the lapsing of a predetermined time period post-activation indicates that certain activities can be resumed or a further dose can be administered.

### BRIEF DESCRIPTION OF THE DRAWINGS

Figure 1 is a diagram of an embodiment of the invention. Therein, the pharmaceutical packaging system (10) comprises a substrate comprising a base surface (1), a blister (2) concave to the base surface containing a dosage form (3), a removable surface (4) layered over the base surface, and an activatable color-changing material (5) between the base surface and the removable surface.

Figure 2 is a diagram of a further embodiment of the invention. Therein, the pharmaceutical packaging system is similar to the embodiment of Figure 1, except that the activatable color-changing material (5) is also layered beneath the removable surface (4) above the blister (2).

Figure 3 is a diagram of a further embodiment of the invention. Therein, the pharmaceutical packaging system is similar to the embodiment of Figure 1, except that the activatable color-changing material (5) is also layered on the inner surface of the blister (2).

Figure 4 is a diagram of a further embodiment of the invention. Therein, the pharmaceutical packaging system is similar to the embodiment of Figure 1, except that the activatable color-changing material (5) is also layered beneath the removable surface (4) above the blister concave (2) and also layered on the inner surface of the blister (2), thereby encasing the dosage form (3).

### DETAILED DESCRIPTION

Pharmaceuticals can cause public safety concerns due to patients not taking the agents as prescribed. One particular concern is the use of opioids to treat pain. A patient under the influence of an opioid for the treatment of pain may not be fully able to safely utilize heavy machinery (e.g., automobile driving). If the patient utilizes heavy machinery during the effective period of the drug, there is a possibility of increased accidents.

Another particular concern is the use of hypnotics to treat insomnia. Insomnia is the inability to sleep when a subject has the opportunity to do so. Insomnia can manifest itself by difficulty falling asleep, or by difficulty falling back to sleep after waking in the middle of the night or after early morning awakenings. An associated public safety issue is when patients utilize sleep aids at a point after the prescribed time during the night, such that the drug continues to cause drowsiness upon awakening in the morning (referred to as "hangover effect"). If the patient utilizes heavy machinery (e.g., automobile driving) during the effective period of the drug, there is a possibility of increased accidents.

As evidence to the extent of this problem, it has been reported by the Stanford University Epidemiology Research Center that approximately 33% of adults in the United States experience middle-of-the-night awakening at least three times each week and that approximately 50% of the adults utilize a sleep aid at least once during the year. There is a particular concern when a sleep aid that is intended to be administered at bedtime (e.g., intended to provide a therapeutic effect for about 8 hours) is administered upon middle-of-the-night awakening. In this case, the drug may continue to provide a sedative effect upon awakening in the morning. There may be a similar concern with sleep aids having a shorter duration of action intended to be administered upon middle-of-the-night awakening, if the drug is administered at a time too close to awakening.

The packaging systems of the present invention include an activatable color-changing material, wherein upon activation, the material changes color after a predetermined time period has elapsed. Utilizing such a packaging system, the patient would be instructed not to participate in certain activities until the color change is effected. This indicator is intended to avoid problems associated with patients resuming certain activities earlier than they should after taking a dose of the drug. The present invention can alternatively be utilized to indicate when a subsequent dose of a pharmaceutical should be administered.

The color-changing material can serve as an indicator for damage to the pharmaceutical packaging system, which may occur during storage, during transport of the system from the manufacturing company to the patient, or during mishandling of the system by the patient himself Upon damaging of the sealed cavity containing the pharmaceutical dosage form, the unintentional activation of the color-changing material could serve as an indicator that the pharmaceutical dosage form itself could also be damaged and should not be used by the patient anymore.

In certain embodiments, the pharmaceutical packaging system of the present invention includes a receptacle comprising an openable seal for accessing the interior of the receptacle; a pharmaceutical dosage form contained in the receptacle; and an activatable color-changing material.

Preferably, the activation of the color-changing material is effected by the opening of the seal for removal of the pharmaceutical dosage form. This is expected to result in increased compliance and use of the invention, as opposed to other packaging systems where removal of the pharmaceutical dosage form and activation of the color-changing material might involve separate steps.

The seal can comprise a base surface layered with the color-changing material and a removable surface layered over the color-changing material, wherein the color-changing material is activated upon separation of the base surface and the removable surface.

Preferably, the base surface and the removable surface are associated with a blister card containing one or more doses of the pharmaceutical dosage form.

The blister may be formed from a rupture-resistant, semi-rigid material. In certain embodiments, the blister is formed of a material selected from the group consisting of plastics, foils and foil-plastic laminations. Suitable materials include polyvinylidene chloride, a combination of polyvinyl chloride/polyethylene/polyvinylidene chloride, pharmaceutical grade polyvinyl chloride, or another thermoplastic material, such as polypropylene, polyethylene, styrene, cold-formed foil, or other suitable materials for packaging. The material may be single ply, multiple ply or laminations. If desired, the material may be selected to retain a desired shape and to be crush resistant. The material or combination of materials should also be chosen to provide barrier protection (e.g., in terms of moisture, oxygen and/or carbon dioxide protection). Such barrier protection may be an important consideration in the present invention as to maintain the integrity of the activatable color change material prior to activation.

The removable seal utilized in the present invention can be formed from a rupture and puncture resistant material wherein the seal is intended to be pealed away from the blister. In other embodiments, the dosage form is intended to be removed from the blister by pushing the dosage form through the seal, wherein the seal can be easily punctured or ruptured. Suitable materials include plastics, paper, foils and combinations thereof Particular materials include polyethylene terephthalate and polypropylene. The degree of puncture resistance can depend on the choice of material and the thickness of the seal.

Further packages and materials therefore, in particular blister packages, which can be used in the context of present application are describe in Soroka, W., "Fundamentals of Packaging Technology", IoPP, 2002, and Yam, K. L., "Encyclopedia of Packaging Technology", John Wiley & Sons, 2009.

In one embodiment, as depicted in Figure 1, the pharmaceutical packaging system (10) can comprises a substrate comprising a base surface (1); a blister (2) concave to the base surface, wherein the blister is adapted to contain a dosage form (3); and a removable surface (4) layered over the base surface, wherein the color-changing material (5) is between the base surface and the removable surface. Typically, the color-changing material is activated upon separation of the base surface and the removable surface.

In another embodiment, as depicted in Figure 2, the pharmaceutical packaging system (10) comprises the activatable color-changing material (5) layered beneath the removable surface (4) above the blister (2). The base surface surrounding the blister may be free of color-changing material (no Fig.), or it may also be layered with color-changing material as depicted in Fig. 2.

In another embodiment, as depicted in Figure 3, the pharmaceutical packaging system (10) also comprises the activatable color-changing material (5) layered on the inner surface of the blister (2). The base surface surrounding the blister may be free of color-changing material (no Fig.), or it may also be layered with color-changing material as depicted in Fig. 3.

In another embodiment, as depicted in Figure 4, the pharmaceutical packaging system (10) comprises the activatable color-changing material (5) layered beneath the removable surface (4) over the blister (2), and layered on the inner surface of the blister (2), thereby encasing the dosage form (3). The base surface surrounding the blister may be free of color-changing material (no Fig.), or it may also be layered with color-changing material as depicted in Fig. 4.

In certain embodiments, a barrier layer can be between the removable surface and the color-changing material and/or between the base surface and the color-changing material. The barrier layer is preferably composed of a material to prevent moisture, oxygen and/or carbon dioxide from contacting the color-changing material during storage. The barrier layer can be selected from the group consisting of polychlorotrifluoroethylene (e.g., Aclar®), polyethylene, polypropylene, polyvinylchloride and a mixture thereof.

In certain embodiments, prior to contact with the blister material, the removable surface material is layered with the color-changing material which is further layered with the barrier layer. This laminar arrangement can be manufactured in a moisture, oxygen and/or carbon dioxide free environment and can be in the form of film roll. After the dosage form is placed into the blister, the layered removable seal is contacted to the blister to enclose the dosage form. This arrangement protects the color-changing material during the packaging process and facilitates manufacture.

A key feature of the pharmaceutical packaging systems as depicted in Figures 1 to 4, is that the patient is able to see color-changing material (5) after opening of the packaging unit and removal of the pharmaceutical dosage form and therefore is able to recognize a visually discernible color change upon the lapsing of a predetermined time period.

The predetermined time period for the color-changing material to change color after activation can be any time that would indicate when a patient, e.g., can resume certain activities or administer a subsequent dose. For example, the time can be from about 1 hour to about 24 hours after activation, from about 4 hours to about 12 hours after administration, or about 8 hours after administration. In certain embodiments, the predetermined time is about 2 hours, about 4 hours, about 6 hours, about 8 hours, about 10 hours, about 12 hours, about 18 hours, or about 24 hours after activation, depending on the particular active agent and dosing regimen. For example, the predetermined time for a color change can be 4 hours or 5 hours, which would be especially suitable for hypnotics having a short duration of action for administration upon middle-of-the-night awakening.

The color-changing material is a material that will effect a color change at a predetermined time subsequent to exposure to environmental air. It is preferably an oxygen-sensitive material and/or a carbon dioxide-sensitive material and/or a moisture sensitive material. In particular, it is sensitive to oxygen and/or carbon dioxide.

In a typical embodiment, the base surface and the removable surface enclose the color-changing material in an environmental air-free environment, in particular an oxygen and/or carbon dioxide and/or moisture free environment. Such environment can, e.g., be a protective gas atmosphere such as N₂. In one embodiment, the base surface and the removable surface enclose the color-changing material in an inert gas atmosphere. The inert gas may be selected from the group consisting of nitrogen, helium, argon, or any other gas or mixture of gases which are commonly used as protective gases. Upon separation of the removable surface from the base surface, the color-changing material is activated by exposure to the surrounding environment (e.g., by exposure to oxygen and/or carbon dioxide and/or moisture). The activated color-changing material will then change color after a predetermined time post-activation. The time post-activation to the color change can be determined by the selection of an appropriate color-changing material or materials, the thickness of the material/materials, and the weight percentages of constituents in the material/materials. In embodiments utilizing moisture-, oxygen-, or carbon dioxide-sensitive material, the manufacture of the packaging system will be performed in suitable conditions so as to maintain the integrity and to avoid the unintended activation of the color-changing material until intended activation. The procedures and materials described in U.S. patent Application Publication No. 2006/0059603; U.S. Patent No. 4,526,752 and U.S. Patent No. 4,812,053 can be modified for utilization in the present invention.

In certain embodiments, an oxygen-sensitive material is used as color-changing material. Said oxygen-sensitive material can be a redox indicator that undergoes a color change upon oxidation. The redox indicator is preferably in reduced form or partially reduced form prior to activation. Redox indicators that can be utilized in the present invention include metal organic complexes and organic redox systems that can be in pH independent or pH dependent form. The indicator can be in reduced form or partially reduced form with the use of a reducing agent, including but not limited to a material selected from the group consisting of bisulfites, hydroxides, cyanides, hydrogen and a combination thereof Specific non-limiting examples of reducing agents are alkali metal bisulfites, ammonium-bisulfites, aliphatic organic compounds, and mixtures thereof

Examples of pH independent redox indictors that undergo a color change from reduced form to oxidized form include Ru complex of 2,2'-bipyridine (yellow to colorless); Fe complex of nitrophenanthroline (red to cyan); N-phenylanthranilic acid (colorless to violet-red); Fe complex of 1,10-phenanthroline (red to cyan); N-ethoxychrysoidine (yellow to red), Fe complex of 2,2'-bipyridine (red to cyan); Fe complex of 5,6-dimethylphenanthroline (red to yellow-green); o-dianisidine (colorless to red), sodium diphenylamine sulfonate (colorless to red-violet), diphenylbenzidine (colorless to violet), diphenylamine (colorless to violet) and viologen (blue to colorless).

Examples of pH dependent redox indictors that undergo a color change from reduced form to oxidized form include sodium 2,6-dibromophenol-indophenol (colorless to blue); sodium 2,6-dibromophenol-indophenol (colorless to blue); sodium o-cresol indophenol (colorless to blue); thionine (colorless to violet), methylene blue (colorless to blue); indigotetrasulfonic acid (colorless to blue); indigotrisulfonic acid (colorless to blue); indigo carmine (colorless to blue); indogomonosulfonic acid (colorless to blue); phenosafranin (colorless to red); safranin T (colorless to red-violet); and neutral red (colorless to red).

The oxygen-sensitive material can be a reaction product of a dye and a decolorant. The dye can include a material such as a triarylmethane. Examples of triarylmethane dyes are brilliant green, malachite green and crystal violet. In other embodiments, the dye can be a thiazone. The decolorant can be a reduing agent as disclosed herein.

In other embodiments, the oxygen-senstive material is gallic acid which converts from colorless to grey upon exposure to oxygen.

In certain embodiments, a carbon dioxide-sensitive material is used as color-changing material. Said carbon dioxide-sensitive material can be a mixture of a pH indicator and a base. The pH indicator can comprise a leuco dye such as phenolphthalein, thymolphthalein, new methylene blue, methyl violet or a mixture thereof. The base can be any suitable material to provide an alkaline environment such as sodium hydroxide.

In certain embodiments, a moisture-sensitive material is used as color-changing material. Said moisture-sensitive material can be cobalt (II) chloride or copper (II) chloride or a mixture thereof

The color change indicating the elapsing of a predetermined time period post-activation can be, e.g., from a first color to a second color, or from a color to colorless, or from colorless to a color, in all cases as long as the color change is readily visually discernible to the untrained eye of the patient. For purposes of the present invention, the color change is also meant to encompass the appearance or disappearance of a symbol. The symbol can be a text message or a warning symbol.

In preferred embodiments, the packaging system of the present invention is utilized with pharmaceuticals that cause sedation, either as a side effect or as intended. In particularly preferred embodiments, the pharmaceutical agent is a sedative/hypnotic agent. Examples of hypnotics that can be utilized in the present invention are zolpidem, zopiclone, eszopiclone, zaleplon, ramelteon, indiplon, pharmaceutically acceptable salts thereof and mixtures thereof

In certain embodiments, the hypnotic agent is zolpidem or a pharmaceutically acceptable salt thereof, such as zolpidem tartrate.

In certain embodiments, the packaging system can include an opioid analgesic dosage form. Opioid analgesics that can be utilized in the present invention include alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl and derivatives, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, sufentanil, tilidine, tramadol, pharmaceutically acceptable salts thereof and mixtures thereof, Preferably, the opioid analgesic is selected from the group consisting of codeine, hydrocodone, hydromorphone, morphine, oxycodone, oxymorphone, tramadol, pharmaceutically acceptable salts thereof and mixtures thereof

The pharmaceutical dosage form may be adapted for one of the following routes of administration: intradermal, intramuscular, intraperitoneal, parenteral, intravenous, subcutaneous, intranasal, epidural, oral, oromucosal, buccal, sublingual, intracerebral, intravaginal, transdermal, rectal, by inhalation, or topical, particularly to the ears, nose, eyes, or skin. In certain embodiments, the pharmaceutical dosage form is in the form of a solid oral dosage form, such as tablets, capsules, gelcaps, caplets, lozenges, granules or powders. The pharmaceutical dosage form may provide immediate release of the active agent, or controlled release of the active agent, or a combination of both.

In certain embodiments, the pharmaceutical packaging system optionally includes a pharmaceutical dosage form, as the present invention is meant to include all intermediates in the production of the final packaging system containing such a dosage form. Such intermediates include but are not limited to the packaging system prior to inclusion of the pharmaceutical dosage from or prior to application of the removable layer.

In other embodiments, the present invention is directed to a kit comprising: (i) a pharmaceutical dosage form (comprising, e.g., an opioid analgesic or sedative/hypnotic); and (ii) an activatable color-changing material, wherein upon activation, the material changes color after a predetermined time has elapsed. In such an embodiment, the color-changing material can be included on any part of the packaging, e.g., as part of a blister card, on a bottle, or on any part of an outer container (e.g., a paper container). In certain embodiments, the color-changing material can be packaged as a discrete unit within the packaging material and not affixed to any particular component.

In certain embodiments, the kit further comprises: (iii) indicia instructing the patient or caregiver that the color change indicates that certain activities can be resumed or a further dose can be administered.

## Claims

1. A pharmaceutical packaging system comprising:
i) a receptacle comprising an openable seal for accessing the interior of the receptacle;
ii) a pharmaceutical dosage form contained in the receptacle; and
iii) an activatable color-changing material, wherein upon activation, the material exhibits a visually discernible color change upon the lapsing of a predetermined time period.

2. The pharmaceutical packaging system of claim 1, wherein the color-changing material is activated upon opening of the seal.

3. The pharmaceutical packaging system of claim 1 or 2,
wherein the seal comprises a base surface layered with the color-changing material and a removable surface layered over the color-changing material, and/or
wherein the seal comprises a removable surface facing the receptacle, which removable surface is layered with the color-changing material; and/or
wherein an inner surface of the interior of the receptacle is layered with the color-changing material.

4. The pharmaceutical packaging system according to any one of the preceding claims, wherein the receptacle comprises:
i) a substrate comprising a base surface (1);
ii) a blister (2) concave to the base surface (1), wherein the blister (2) is adapted to contain the pharmaceutical dosage form (3); and
iii) a removable surface (4) layered over the base surface (1), wherein the activatable color-changing material (5) is between the base surface and the removable surface and/or is beneath the removable surface above the blister and/or is layered on the inner surface of the blister.

5. The pharmaceutical packaging system according to claim 3 or 4, further comprising a barrier layer between the removable surface and the color-changing material and/or between the base surface and the color-changing material.

6. The pharmaceutical packaging system according to any one of claims 3 to 5, wherein the color-changing material is activated upon separation of the base surface and the removable surface.

7. The pharmaceutical packaging system according to any one of the preceding claims, wherein the predetermined time period is from about 1 hour to about 24 hours, preferably from about 4 to about 12 hours, more preferably from about 4 hours to about 5 hours after activation.

8. The pharmaceutical packaging system according to any one of the preceding claims, wherein the color-changing material is an oxygen-sensitive material and/or a carbon dioxide-sensitive material and/or a moisture -sensitive material.

9. The pharmaceutical packaging system according to claim 8, wherein the color-changing material is enclosed in an oxygen-free and/or in a carbon dioxide-free and/or in a moisture-free environment.

10. The pharmaceutical packaging system according to claim 8 or 9, wherein the oxygen-sensitive material is a redox indicator that undergoes a color change upon oxidation.

11. The pharmaceutical packaging system according to claim 8 or 9, wherein the oxygen-sensitive material is a reaction product of a dye and a decolorant.

12. The pharmaceutical packaging system of claim 11, wherein the dye comprises a triarylmethane dye or a thiazone dye.

13. The pharmaceutical packaging system according to claim 11 or 12, wherein the decolorant is a reducing agent, which preferably is selected from the group consisting of bisulfites, hydroxides, cyanides, hydrogen and a combination thereof, more preferably is selected from the group consisting of alkali metal bisulfites, ammonium-bisulfites, aliphatic organic compounds, and mixtures thereof.

14. The pharmaceutical packaging system according to claim 8 or 9, wherein the carbon dioxide-sensitive material is a mixture of a pH indicator and a base,
i) wherein preferably the pH indicator comprises a leuco dye, which is more preferably selected from the group consisting of phenolphthalein, thymolphthalein, new methylene blue, methyl violet, and mixtures thereof; and/or
ii) wherein preferably the base comprises sodium hydroxide.

15. The pharmaceutical packaging system according to any one of the preceding claims, wherein the pharmaceutical dosage form comprises
i) a sedative/hypnotic agent, which is preferably selected from the group consisting of zolpidem, zopiclone, eszopiclone, zaleplon, ramelteon, indiplon and pharmaceutically acceptable salts thereof, and more preferably is zolpidem or a pharmaceutically acceptable salt thereof; and/or
ii) an opioid analgesic, which is preferably selected from the group consisting of codeine, hydrocodone, hydromorphone, morphine, oxycodone, oxymorphone, tramadol, or a pharmaceutically acceptable salt thereof.

16. A pharmaceutical packing system according to any one of the preceding claims for use in the treatment of a patient suffering from a condition, wherein preferably the condition is selected from the group consisting of pain and insomnia.
